(19) Europäisches Patentamt
European Patent Office
Office européen des brevets

(11) Publication number: 0 308 913 A2

# (12) EUROPEAN PATENT APPLICATION

(21) Application number: 88115533.7

(22) Date of filing: 22.09.88

(51) Int. Cl.4: C07D 471/08 , A61K 31/435 , C07D 221/22

Claims for the following Contracting States: ES + GR.

(30) Priority: 24.09.87 US 101716

(43) Date of publication of application: 29.03.89 Bulletin 89/13

(84) Designated Contracting States: AT BE CH DE ES FR GB GR IT LI LU NL SE

(71) Applicant: HOECHST-ROUSSEL PHARMACEUTICALS INCORPORATED
Route 202-206 North
Somerville New Jersey 08876(US)

(72) Inventor: Allen, Richard C.
RD 3, Box 514 Hillcrest Road
Flemington, NJ 08822(US)
Inventor: Wettlaufer, David G.
9 South Auten Street
Somerville, NJ 08876(US)

(74) Representative: Becker, Heinrich Karl Engelbert, Dr. et al
HOECHST AKTIENGESELLSCHAFT Central Patent Department P.O. Box 80 03 20
D-6230 Frankfurt am Main 80(DE)

## (54) 2,6-Methanopyrrolo-3-benzazocines, a process and intermediates for their preparation and their use as medicaments.

(57) The present invention relates to novel 2,6-methanopyrrolo-3-benzazocines, and a process and intermediates for their preparation. The compounds have analgesic activity and can, therefore, be used as medicaments.

EP 0 308 913 A2

## 2,6-Methanopyrrolo-3-benzazocines, a process and intermediates for their preparation and their use as medicaments

This invention relates to 2,6-methanopyrrolo-3-benzazocines of the formula

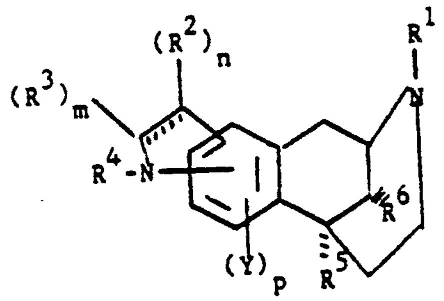

Formula I

wherein $R^1$ is selected from the group consisting of hydrogen, loweralkyl, loweralkenyl, cycloalkylloweralkyl, arylloweralkyl and -C(O)$R^9$ where $R^9$ is hydrogen, loweralkyl, or loweralkoxy; $R^2$ and $R^3$ are independently selected from the group consisting of loweralkyl, arylloweralkyl, halogen, oxo, and -C(O)$R^7$ wherein $R^7$ is selected from the group consisting of hydrogen, loweralkyl, aryl, arylloweralkyl, hydroxy, loweralkoxy, arylloweralkoxy, aryloxy, and amino; $R^4$ is selected from the group consisting of hydrogen, loweralkyl, arylloweralkyl and -C(O)$R^8$ wherein $R^8$ is selected from the group consisting of hydrogen, arylloweralkyl, aryl, loweralkyl, loweralkoxy, arylloweralkoxy, and aryloxy; $R^5$ and $R^6$ are independently hydrogen or loweralkyl, or taken together are a bivalent radical of the formula $-(CH_2)_4-$; m, n, and p are integers independently having values of zero or 1 with the proviso that the sum of m and n only exceeds 1 when at least one of $R^2$ and $R^3$ is linear loweralkyl, halogen, or oxo; Y is halogen or loweralkyl; and the dotted line in said compound is an optional bond; the geometrical isomers, optical antipodes or pharmaceutically acceptable acid addition salts thereof, which, alone or in combination with inert adjuncts, are useful in alleviating pain.

Of particular interest are 2,6-methanopyrrolo-3-benzazocines of the formulas:

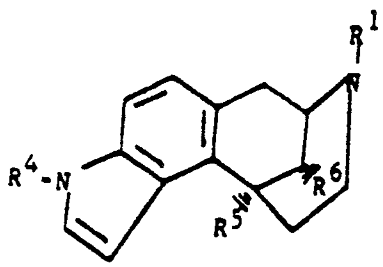

Formula IIa

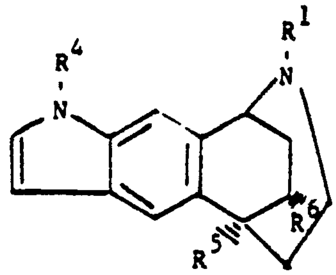

Formula IIb;

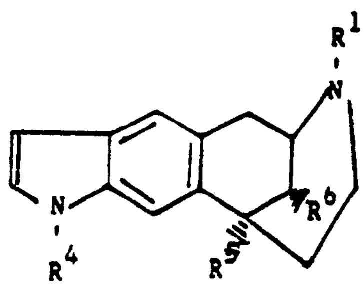

Formula IIc; and

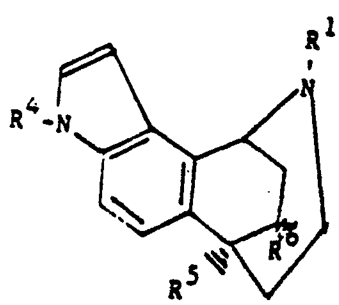

Formula IId

Subgeneric to the 2,6-methanopyrrolo-3-benzazocines of this invention are Formula I compounds wherein:

(a) $R^1$ is hydrogen;

(b) $R^1$ is loweralkyl;

(c) $R^1$ is cycloalkylloweralkyl;

(d) $R^1$ is loweralkenyl;

(e) $R^1$ is arylloweralkyl;

(f) $R^1$ is $-C(O)R^9$ wherein $R^9$ is as previously defined;

(g) $R^2$ and $R^3$ are independently selected from the group consisting of loweralkyl, aryloweralkyl, halogen, oxo, and $-C(O)R^7$ as previously defined;

(h) $R^2$ and $R^3$ are oxo;

(i) $R^4$ is hydrogen;

(j) $R^4$ is loweralkyl;

(k) $R^4$ is arylloweralkyl;

(l) $R^4$ is $-C(O)R^8$ as previously defined;

(m) $R^5$ and $R^6$ are independently hydrogen or loweralkyl;

(n) $R^5$ and $R^6$ taken together are a bivalent radical of the formula $-(CH_2)_4-$; and

(o) p is zero.

In a further embodiment this invention relates to intermediates of the formula

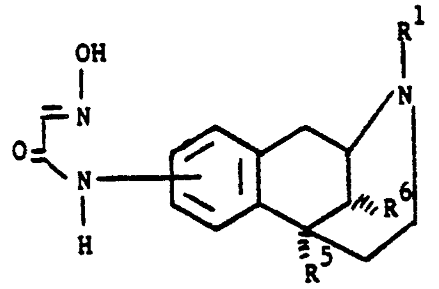

Formula 3

wherein $R^1$ is selected from the group consisting of hydrogen, loweralkyl, loweralkenyl, cycloalkylloweralkyl, arylloweralkyl and -C(O)$R^9$ wherein $R^9$ is hydrogen, loweralkyl or loweralkoxy; and $R^5$ and $R^6$ are independently hydrogen or loweralkyl, or taken together are a bivalent radical of the formula $-(CH_2)_4-$, which have utility in the production of the hereinbeforementioned 2,6-methanopyrrolo-3-benzazocines.

As used throughout the specification and appended claims, the following definitions shall apply:

"Loweralkyl" - a linear or branched, acyclic hydrocarbon radical containing no unsaturation and having the formula $-C_xH_{2x+1}$ wherein x is an integer having a value of 1 to 7 inclusive, such as methyl, ethyl, 1-propyl, 2-propyl, 1-butyl, 1-pentyl, 2-pentyl, 3-hexyl, 4-heptyl, and the like. Preferred loweralkyls are those radicals wherein x has a value of 1 to 3 inclusive, most preferably 1 or 2.

"Loweralkenyl" - a linear or branched, acyclic hydrocarbon radical having one olefinic bond and represented by the formula: $-C_xH_{2x-1}$, wherein x is an integer having a value of 3 to 7 inclusive, such as 2-propenyl, 3-butenyl, 3-pentenyl, 3-hexenyl, 6-heptenyl, and the like. Preferred loweralkenyls are those radicals wherein x has a value of 3 to 5 inclusive, and, most preferably, is 3.

"Cycloalkyl" - a cyclic hydrocarbon radical of the formula - $C_xH_{2x-1}$ wherein x is an integer having a value of 3 to 7 inclusive, such as cyclopropyl, cyclobutyl, cyclopentyl, and cycloheptyl. Preferred cycloalkyls are those radicals wherein x has a value of 3 to 6 inclusive, and, most preferably is 3.

"Cycloalkylloweralkyl" - a loweralkyl group having a cycloalkyl substituent thereon.

"Loweralkoxy" - an acyclic organic radical of the formula $-OC_xH_{2x+1}$ wherein x is an integer having a value of 1 to 7 inclusive, such as methoxy, ethoxy, 1- and 2-propoxy, 1,2-dimethylethoxy, 1-butoxy, 1- and 2-pentoxy, 3-hexoxy, 4-heptoxy and the like. Preferred loweralkoxys are those radicals wherein x has a value of 1 to 5 inclusive, most preferably, 1 to 3 inclusive.

"Aryl" - a phenyl group optionally substituted by up to 3 substituents each of which is independently loweralkyl, loweralkoxy, halogen, trifluoromethyl, nitro or cyano.

"Loweralkoxycarbonyl" - an acylic organic radical of the formula $-C(O)OC_xH_{2x+1}$ wherein x is an integer having a value from 1 to 5 inclusive, such as methoxycarbonyl, ethoxycarbonyl, 1- and 2-propoxycarbonyl, 1-butoxycarbonyl, 1- and 2-pentoxycarbonyl and the like. Preferred loweralkoxycarbonyls are those radicals wherein x has a value of 1 to 4 inclusive, and most preferably, is 1 or 2.

"Halogen" - a member of the group consisting of fluorine, chlorine, bromine or iodine radicals.

"Arylloweralkyl" - a loweralkyl group having an aryl substituent thereon.

"Aryloxy" - a monovalent radical which consists of an aryl group linked through an ether oxygen and having its free valence bond from the ether oxygen.

The 2,6-methanopyrrolo-3-benzazocines of this invention are synthesized by the process illustrated in the Reaction Schemes which follow.

To prepare the parent system, i.e., Formula II, 2,6-methanopyrrolo-3-benzazocine diones, a nitro-substituted 1,2,3,4,5,6-hexahydro-2,6-methano-3-benzazocine 1 is reduced to the corresponding amino-substituted benzazocine 2, which is converted to an isonitrosoacetanilide 3, which in turn is cyclized to an isatin 4. See Reaction Scheme A.

The preparation of nitro-substituted 1,2,3,4,5,6-hexahydro-2,6-methano-3-benzazocines is well known in the art. Conventional preparations include the nitration of 1,2,3,4,5,6-hexahydro-2,6-methano-3-benzazocines by treatment with nitric acid in an appropriate solvent (e.g. glacial acetic acid). Alkylation of the nitro substituted secondary benzazocine with a halide of the formula $R^1X$ wherein $R^1$ is loweralkyl, loweralkenyl, cycloalkylloweralkyl, arylloweralkyl or -C(O)$R^9$ as previously described, and X is halogen, preferably

chlorine or bromine, provides a nitro-substituted benzazocine having a group $R^1$ at the nitrogen atom in the 3-position of the benzazocine ring. Where $R^1$ is methyl, removal of the methyl group to give the secondary amine is achieved by reaction with an appropriate haloformate such as, for example, 1-chloroethyl chloroformate, followed by methanolysis. The alkylation of the nitro-substituted benzazocine is ordinarily conducted in an inert organic solvent in the presence of an appropriate acid acceptor (e.g. an alkali metal carbonate and/or bicarbonate such as, for example, sodium carbonate, sodium bicarbonate, potassium carbonate, potassium bicarbonate, and the like). Suitable solvents include polar aprotic solvents such as dimethylformamide, dimethylacetamide, dimethylsulfoxide, and the like, alkanols such as propanol and n-butanol; and acetone. Dimethylformamide is preferred. See, for example, U. S. Patent Nos. 4,255,579, 4,127,577 and 4,032,529 all assigned to Sterling Drug Inc., setting forth the nitration and subsequent alkylation of 1,2,3,4,5,6-hexahydro-2,6-methano-3-benzazocines in greater detail.

Reduction of the nitro-substituted benzazocine 1 is also accomplished by conventional methods which include both catalytic hydrogenation in the presence of a noble metal catalyst (e.g. platinum, rhodium, palladium, and the like) and chemical reduction. Catalytic hydrogenation is ordinarily conducted in the presence of an alkanol solvent (e.g. methanol, ethanol, 1- and 2-propanol, and the like, and mixtures thereof) at a temperature of from about 20° C to about 75° C and a hydrogen gas pressure of from about 16 psig to about 65 psig. Preferably, catalytic hydrogenation is conducted at a temperature of from about 20° C to about 30° C and a hydrogen gas pressure of from about 50 psig to about 60 psig, in the presence of methanol employing 5% palladium on barium sulfate as the catalyst. Chemical reduction of the nitro-substituted benzazocine may be achieved utilizing a metal (e.g. iron) and a mineral acid (e.g. hydrochloric acid) as described more fully in the above cited U. S. Patents assigned to Sterling Drug Inc.

The conversion of the amino-substituted benzazocine 2 to an isatin 4 is similarly accomplished utilizing techniques which are well known in the art. See, for example, the procedures described in A. A. Asselin et al., J. Med. Chem. 29, 648 (1986). Typically, the amino-substituted benzazocine 2 is treated with hydroxylamine hydrochloride and chloral hydrate in the presence of an anhydrous alkali metal sulfate (e.g. potassium sulfate, sodium sulfate, and the like, sodium sulfate being preferred). The reaction is ordinarily conducted in the presence of water at a temperature of from about 80° C to the reflux temperature of the solvent medium, preferably at reflux. Cyclization of the resulting isonitrosoacetanilide 3 is achieved by treatment with an appropriate acid (e.g. mineral acids such as hydrochloric, sulfuric, and phosphoric acid, aqueous sulfuric acid being preferred) at a temperature of from about 70° C to about 85° C, preferably from about 70° C to about 75° C, to yield the isatin 4.

The reduction of the parent 2,6-methanopyrrolo-3-benzazocine dione system to hexahydro- and octahydro-2,6-methanopyrrolo-3-benzazocines is illustrated in Reaction Scheme B. As illustrated, reduction of an isatin 6 wherein $R^1$ is loweralkyl provides the corresponding hexahydro- and octahydro-2,6-methanopyrrolo-3-benzazocines 7 and 8. Reduction to the hexahydro-2,6-methanopyrrolo-3-benzazocine 7 is ordinarily conducted in the presence of an inert organic solvent (e.g. ethereal solvents such as dioxane, diethyl ether, tetrahydrofuran, and the like; tetrahydrofuran being preferred) utilizing a reducing agent such as lithium aluminium hydride or borane. The reduction is ordinarily carried out at a temperature of from about 0° C to the reflux temperature of the solvent medium, preferably at reflux. Desirably the reduction is conducted under an inert atmosphere. It should be noted that the reduction of isatins 5 wherein $R^1$ is a radical of the formula -C(O)$R^9$ as previously described can result in the simultaneous reduction of the acyl group, providing an alternative mechanism for the synthesis of loweralkyl-substituted benzazocines 7.

Further reduction of hexahydro-2,6-methanopyrrolo-3-benzazocines 7 by treatment with a reducing agent such as, for example, an alkali metal organoborohydride such as sodium cyanoborohydride yields the corresponding octahydro-2,6-methanopyrrolo-3-benzazocine 8. This subsequent reduction is ordinarily conducted at a reduced temperature of from about 15° C to about 20° C in the presence of an alkanoic acid (e.g. formic, acetic, 1- and 2-propionic acid, and the like, acetic acid being preferred).

2,6-Methanopyrrolo-3-benzazocines wherein $R^1$ is hydrogen may be furnished by cleaving a loweralkyl-substituted benzazocine 9 protected at the nitogen atom of the pyrrolo ring to an unsubstituted benzazocine 10, and then removing the protecting group of 10 to afford the benzazocine 11.

Protection of the pyrrolo nitrogen atom is achieved by conventional manipulation techniques. For example, a loweralkyl-substituted benzazocine 7 may be reacted with a strong base (e.g. an alkali metal hydride such as sodium hydride, or potassium hydride, or lithium bis(trimethylsilyl)amide) in a suitable solvent medium (e.g. a polar aprotic solvent such as dimethylformamide, dimethylsulfoxide, dimethylacetamide, hexamethylphosphoramide, and the like or an ethereal solvent such as tetrahydrofuran) at a temperature of from about -80° C to about 80° C, and then treated with an arylloweralkylsulfonyl halide or arylsulfonylhalide (e.g. benzenesulfonyl chloride, toluene-sulfonyl chloride, and the like) or triisopropylsilyltrifluoromethane sulfonate to give the protected benzazocine 9.

Cleavage of the 3-loweralkyl group is likewise effected by conventional synthetic techniques. For example, the protected benzazocine 9 may be treated with an alkyl-, phenyl- or benzyl- chloroformate (e.g. ethyl chloroformate, 1-chloroethyl chloroformate, phenyl chloroformate, benzyl chloroformate, and the like) and the resulting carbamate hydrolyzed to the unsubstituted benzazocine 10. Optionally, the chloroformate treatment is conducted in the presence of an alkali metal carbonate and/or bicarbonate such as, for example, sodium carbonate, potassium carbonate, sodium bicarbonate, potassium bicarbonate, and the like. Hydrolysis of the carbamate intermediate may be achieved by treatment with a suitable base (e.g. an alkali metal hydroxide such as, for example, potassium, sodium, and lithium hydroxide) in an aqueous alkanol (e.g. methanol, ethanol, 1- and 2-propanol and the like) at a temperature of from about 25° to the reflux temperature of the solvent medium, or in the case of the 1-chloroethyl carbamate by methanolysis.

Well known techniques are similarly available for removal of the protecting group at the nitrogen atom of the pyrrolo ring. For example, refluxing the protected benzazocine 10 in the presence of an alkali metal aluminum hydride (e.g. lithium aluminum hydride, sodium bis (2-methoxyethoxy)aluminum hydride, and the like) in an appropriate solvent medium (e.g. ethereal solvents such as, for example, tetrahydrofuran, dimethoxyethane, bis(2-methoxyethyl)ether, diethyl ether, and dioxane) or, in the case of the triisopropylsilyl group, treatment with tetra-n-butyl-ammonium fluoride in tetrahydrofuran. The resultant hexahydro-2,6-methanopyrrolo-3-benzazocine 11 may be reduced as previously described in the context of loweralkyl-substituted hexahydro-2,6-methanopyrrolo-3-benzazocines to the corresponding octahydrobenzazocine 12.

2,6-Methanopyrrolo-3-benzazocines 14 wherein $R^1$ is loweralkyl, loweralkenyl, cycloalkylloweralkyl or arylloweralkyl may be produced by the alkylation of unsubstituted benzazocines 10 followed by deprotection of the resulting substituted benzazocine 13. Alkylation may be achieved, for example, by treating the unsubstituted benzazocine 10 with a halide of the formula $R^1Z$, wherein Z is chlorine, bromine or iodine and $R^1$ is as previously described, in the presence of a base suspended or dissolved in a suitable solvent. Suitable bases include alkali metal carbonates and bicarbonates such as, for example, sodium carbonate, sodium bicarbonate, potassium carbonate, potassium bicarbonate, and the like. Among the suitable solvents there may be mentioned polar aprotic solvents, chlorinated hydrocarbons, ethereal solvents, and the like, such as for example, dimethylformamide, dimethylacetamide, bis(2-methoxyethyl ether), dimethoxyethane, dimethylsulfoxide, hexamethylphosphoramide, dichloromethane, diethyl ether, tetrahydrofuran, dioxan, and the like. The alkylation is ordinarily conducted at temperatures of from about 20° C to reflux. Deprotection of 3-substituted benzazocines 13 and reduction of the corresponding hexahydro- and oxtahydro-derivatives 14 and 15 is as previously described.

The sequence of protection, alkylation and deprotection steps described in the context of the hexahydrobenzazocines of this invention is equally applicable to octahydrobenzazocines. Included among the compounds of this invention are the following:

3-benzyl-1,2,3,4,5,6-hexahydro-6,12-dimethyl-2,6-methanopyrrolo[3,2-h][3]-benzazocine-7,8(9H)-dione;
3-benzyl-1,2,3,4,5,6-hexahydro-6,12-dimethyl-2,6-methano-9H-pyrrolo[3,2-h][3]-benzazocine;
3-benzyl-1,2,3,4,5,6-octahydro-6,12-dimethyl-2,6-methano-9H-pyrrolo[3,2-h][3]benzazocine;
1,2,3,4,5,6,7,8-octahydro-6,12-dimethyl-3-(2-propenyl)-2,6-methano-9H-pyrrolo[3,2-h][3]benzazocine;
1,2,3,4,5,6,7,8-hexahydro-6,12-dimethyl-3-(2-propenyl)-2,6-methanopyrrolo[3,2-h][3]benzazocine-7,8(9H)-dione;
3-cyclopropylmethyl-1,2,3,4,5,6-hexahydro-6,12-dimethyl-2,6-methano-9H-pyrrolo[3,2-h][3]benzazocine;
3-cyclopropylmethyl-1,2,3,4,5,6-hexahydro-6,12-dimethyl-2,6-methanopyrrolo[3,2-h][3]benzazocine-7,8(9H)-dione;
3-cyclopropylmethyl-1,2,3,4,5,6,10,11-octahydro-6,12-dimethyl-2,6-methano-9H-pyrrolo[2,3-j][3]benzazocine;
1,2,3,4,5,6-hexahydro-3,6,12-trimethyl-2,6-methano-8H-pyrrolo[2,3-i][3]benzazocine;
1,2,3,4,5,6-hexahydro-3,6,12-trimethyl-2,6-methanopyrrolo[2,3-i][3]benzazocine-9,10(8H)-dione;
1,2,3,4,5,6-hexahydro-3,6,7,12-tetramethyl-2,6-methanopyrrolo[2,3-i][3]benzazocine-9,10(8H)-dione;
1,2,3,4,5,6-hexahydro-3,6,7,12-tetramethyl-2,6-methano8H-pyrrolo[2,3-i][3]benzazocine;
11-acetyl-1,2,3,4,5,6-hexahydro-3,6,12-trimethyl-2,6-methano-9H-pyrrolo[2,3-j][3]benzazocine;
11-aminocarbonyl-1,2,3,4,5,6-hexahydro-3,6,12-trimethyl-2,6-methano-9H-pyrrolo[2,3-j][3]benzazocine;
1,2,3,4,5,6-hexahydro-3,6,12-trimethyl-2,6-methano-8H-pyrrolo[2,3-i][3]benzazocine;
1,2,3,4,5,6-hexahydro-3,6,12-trimethyl-2,6-methanopyrrolo[2,3-i][3]benzazocine-9,10(8H)-dione;
1,2,3,4,5,6,10,11-octahydro-3,6,12-trimethyl-2,6-methano-9H-pyrrolo[2,3-j][3]benzazocine;
3-benzyl-1,2,3,4,5,6-hexahydro-6,12-dimethyl-2,6-methano-8H-pyrrolo[2,3-i][3]benzazocine;
7-chloro-1,2,3,4,5,6-hexahydro-3,6,12-trimethyl-2,6-methanopyrrolo[2,3-j][3]benzazocine-10,11(9H)-dione;
1,2,3,4,5,6-hexahydro-3,6,12-trimethyl-2,6-methano-9H-pyrrolo[3,2-h][3]benzazocine;
1,2,3,4,4a,5,6,8-octahydro-14-methyl-5,11b-(iminoethano)-11bH-naphth[1,2-f]indole;
1,2,3,4,5,6,7,10-octahydro-14-methyl-5,11b-(iminoethano)-11bH-naphth[2,1-f]indole;

3,6,7,7a,8,9,10,11-octahydro-14-methyl-7,11a-(iminoethano)-11aH-naphth[1,2-e]indole;
1,4,5,5a,6,7,8,9-octahydro-14-methyl-5,9a-(iminoethano)-9aH-naphth[2,1-e]indole;
decahydro-14-methyl-5-11b-(iminoethano)-11bH-naphth[2,1-f]indole;
3,6,7,7a,8,9,10,11-octahydro-14-(2-phenylethyl)-7,11a-(iminoethano)-11aH-naphth[1,2-e]indole;
1,2,3,4,5,6-hexahydro-6,12-dimethyl-3-(2-phenylethyl)-2,6-methano-9H-pyrrolo[3,2-h][3]benzazocine;
1,2,3,4,5,6-hexahydro-6,12-dimethyl-3-(3-methyl-2-butenyl)-2,6-methano-10H-pyrrolo[3,2-i][3]benzazocine;
3-cyclopropylmethyl-1,2,3,4,5,6,9,10-octahydro-6,12-dimethyl-2,6-methano-8H-pyrrolo[2,3-i][3]benzazocine;
1,2,3,4,5,6-hexahydro-6,12-dimethyl-3-(3-methyl-2-butenyl)-2,6-methano-8H-pyrrolo[2,3-i][3]benzazocine;
1,2,3,4,5,6,10,11-octahydro-6,12-dimethyl-3-(2-phenylethyl)-2,6-methano-9H-pyrrolo[2,3-j][3]benzazocine;
1,2,3,4,5,6-hexahydro-6,12-dimethyl-3-(2-phenylethyl)-2,6-methano-10H-pyrrolo[3,2-i][3]benzazocine;
6,12-diethyl-1,2,3,4,5,6-hexahydro-3-methyl-2,6-methano-8H-pyrrolo[2,3-i][3]benzazocine;
6,12-diethyl-1,2,3,4,5,6-hexahydro-3-methyl-2,6,-methano-9H-pyrrolo[3,2-h][3]benzazocine;
6,12-diethyl-1,2,3,4,5,6-hexahydro-3-methyl-2,6-methano-10H-pyrrolo[3,2-i][3]benzazocine;
6,12-diethyl-1,2,3,4,5,6-hexahydro-3-methyl-2,6-methano-9H-pyrrolo[2,3-j][3]benzazocine;
1,2,3,4,5,6-hexahydro-3-methyl-2,6-methano-9H-pyrrolo[3,2,-h][3]benzazocine;
1,2,3,4,5,6-hexahydro-3-methyl-2,6-methano-10H-pyrrolo[3,2,-i][3]benzazocine;
1,2,3,4,5,6-hexahydro-6,12-dimethyl-3-(2-phenylethyl)-2,6-methano-8H-pyrrolo[3,2,-i][3]benzazocine;
1,2,3,4,5,6-hexahydro-6,12-dimethyl-3-(3-methyl-2-butenyl)-2,6-methano-9H-pyrrolo[3,2-h][3]benzazocine;

The 2,6-methanopyrrolo-3-benzazocines of this invention are useful as analgetics due to their ability to alleviate pain in mammals. The procedure employed to determine analgetic utility is a modification of the phenyl-p-quinone writhing assay in mice, a standard assay for analgesia [Proc. Soc. Exptl. Bio. Med., 95 729 (1957)]. In the modified procedure phenyl-p-benzoquinone (Eastman, 12.5 mg) is dissolved in 5 ml of 95% ethanol and the solution is diluted to a total volume of 100 ml with distilled water. The solution is administered to the subject mice subcutaneously at a dose of 10 ml per kg of body weight. A characteristic "writh", an inward rotation of one or more feet with twisting and turning of the trunk, drawing in of the abdominal wall, lordosis and arching of the back, is produced.

A total of 28 male mice (Charles River, CD-1), weighing 18 to 30 grams, are employed for a time response. The subject animals receive food and water ad libitum. Test compounds are dissolved in distilled water, or suspended in distilled water containing one drop of a suitable surfactant, such as Tween-80.

Four groups of five animals (20 animals) are given the test compound subcutaneously at 15, 30, 45 and 60 minutes prior to administration of the phenyl-p-quinone. Four control groups of 2 animals (8 animals) receive an equal volume of the vehicle. After the administration of the phenyl-p-quinone, the mice are placed separately in one liter beakers, and after five minutes, are observed for ten minutes. The number of writhes for each animal is recorded. The following formula is used to compute the percent inhibition:

$$\frac{\bar{x}\text{ Writhes in Control Group} - \bar{x}\text{ Writhes in Drug Group}}{\bar{x}\text{ Writhes in Control Group}} \times 100$$

The time period with the greatest percent of inhibition is considered the peak time. A dose range determination is generally reserved for those compounds which inhibit writhing by greater than 65-70% at the screening dose.

A dose range determination is run in the same manner as the time response except 10 animals per group are tested at the peak time of test drug activity. Fifty animals, 4 test drug groups, and a vehicle control are employed. Animals are dosed and tested in a randomized manner. A calculated $ED_{50}$, i.e., the dose at which 50% inhibition of writhing is produced, is determined by a computer linear regression analysis. The calculated subcutaneous (s.c.) dose effecting an approximately 50% inhibition of writhing ($ED_{50}$) in mice produced in this assay is as follows:

| Compound | Analgesic Activity (Inhibition of Writhing ED (mg/kg, s.c.) |
|---|---|
| 1,2,3,4,5,6-hexahydro-3,6,12-trimethyl-2,6-methano-8H-pyrrolo[2,3-i][3]benzazocine | 29.5 |
| 1,2,3,4,5,6-hexahydro-3,6,12-trimethyl-2,6-methano-9H-pyrrolo[2,3-j][3]benzazocine | 3Ø.4 |
| 1,2,3,4,5,6,9,1Ø-octahydro-3,6,12-trimethyl-2,6-methano-8H-pyrrolo[2,3-i][3]benzazocine | 35.5 |
| 1,2,3,4,5,6-hexahydro-3,6,12-trimethyl-2,6-methanopyrrolo[2,3-j][3]benzazocine-1Ø,11(9H)-dione | 24.3 |
| 1,2,3,4,5,6-hexahydro-3,16,12-trimethyl-2,6-methano-9H-pyrrolo-[3,2-h][3]-benzazocine | Ø.6 |
| 1,2,3,4,5,6-hexahydro-3,6,12-trimethyl-2,6-methano-1ØH-pyrrolo[3,2-i][3]-benzazocine | 1.8 |
| pentazocine | 1.3 |

Analgesia production is achieved when the 2,6-methanopyrrolo-3-benzazocines of this invention are administered to a subject requiring such treatment as an effective oral, parenteral or intravenous dose of from 0.1 to 50 mg/kg of body weight per day. 2,6-Methanopyrrolo-3-benzazocines which achieve effective analgesia production at does of about 5 mg/kg of body weight per day are particularly desirable. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

Effective amounts of the present invention may be administered to a subject by any one of various methods, for example, orally as in capsules or tablets, parenterally in the form of sterile solutions or suspensions, and in some cases intravenously in the form of sterile solutions. 2,6-Methanopyrrolo-3-benzazocines of this invention, while effective themselves, may be formulated and administered in the form of their pharmaceutically acceptable addition salts for purposes of stability, convenience or crystallization, increased solubility and the like.

Preferred pharmaceutically acceptable addition salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and the like, and salts of tribasic carboxylic acids such as, for example, carboxysuccinic acid, citric acid and the like.

Effective quantities of the compounds of this invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0 and 300 milligrams of the active compound.

The tablets, pills, capsules, troches and the like may also contain the following ingredients: a binder such as microcrystalline cellulose, gum tragancanth or gelatin; an excipient such as starch or lactose, a disintegrating agent such as alginic acid, PromogelTM, cornstarch and the like; a lubricant such as magnesium stearate or Sterotex; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the preceeding type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and nontoxic in the amounts used.

For the purpose of parenteral therapeutic administration, the active compounds of this invention may be incorporated into a solution or suspension. These preparations should contain at least 0.1% of active compound, but may be varied between 0.5 and about 50% of the weight thereof. The amount of active compounds in such compositions is such that a suitable dosage will be obtained. Preferred compositions and preparations according to the present invention are prepared so that a parenteral dosage unit contains between 0.5 and 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzylalcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes of multiple dose vials made of glass or plastic.

## Examples

The following examples are for illustrative purposes only and are not to be construed as limiting the invention.

Example 1

1,2,3,4,5,6-Hexahydro-3,6,12-trimethyl-2,6-methano-8H-pyrrolo[2,3-i][3]benzazocine

Step 1

A solution of 21.0 g of 3-ethoxycarbonyl-1,2,3,4,5,6-hexahydro-6,11-dimethyl-8-nitro-2,6-methano-3-benzazocine and 500 ml of methanol was hydrogenated over 5% palladium on barium sulfate (1.00 g) at 3,85 bar. Upon completion of the reaction, the solution was filtered free of solids and concentrated. The concentrate was dissolved in 300 ml of 5% aqueous hydrochloric acid and 370 ml of water, and treated with 59.3 g of anhydrous sodium sulfate and 14.5 g of hydroxylamine hydrochloride. After heating to reflux, the resulting solution was treated with a refluxing solution of 13.1 g of chloral hydrate and 89 ml of water. The reaction mixture was refluxed for an additional hour, cooled to 0°C, and basified to a pH of 8-9 by the addition of dilute aqueous ammonium hydroxide. The mixture was extracted with dichloromethane-ethyl acetate, washed with brine, dried over anhydrous magnesium sulfate and concentrated. The concentrate was dissolved in 432 ml of 10% aqueous sulfuric acid, warmed for 25-35 min in an oil bath (75-78°C), poured over ice, diluted with water, and extracted with dichloromethane. The extract was washed with brine, dried over anhydrous magnesium sulfate, filtered, and concentrated. The concentrate was purified by flash chromatography on silica gel, eluting with ethyl acetate/hexane to yield, as a major product, 3-ethoxycarbonyl-1,2,3,4,5,6-hexahydro-6,12-dimethyl-2,6-methanopyrrolo[2,3-i]-[3-benzazocine-9,10(8H)-dione and, as a minor product, 3-ethoxycarbonyl-1,2,3,4,5,6-hexahydro-6,12-dimethyl-2,6-methanopyrrolo-[3,2-h][3]benzazocine-7,8-(9H)-dione.

Step 2

To a stirred slurry of 2.92 g of lithium aluminum hydride in 85 ml of tetrahydrofuran, cooled to 0°C under nitrogen, was added, via cannula, a solution of 1.00 g of 3-ethoxy-carbonyl-1,2,3,4,5,6-hexahydro-6,12-dimethyl-2,6-methanopyrrolo[2,3-i][3]benzazocine-9,10(8H)-dione in 25 ml of tetrahydrofuran. The reaction mixture was refluxed for 3 hours, cooled to 0°C, and quenched with 28 ml of 10% aqueous tetrahydrofuran.

The aqueous mixture was then dried over anhydrous sodium sulfate, filtered, and concentrated. The concentrate was purified by means of flash chromatography (silica gel; utilizing as the eluent 2% triethylamine/0-4% methanol/ethylacetate) to yield 0.50 g (67%) of 1,2,3,4,5,6-hexahydro-3,6,12-trimethyl-2,6-methano-8H-pyrrolo[2,3-i][3]benzazocine.

Recrystallization from diethyl ether afforded the analytical sample, m.p. 150-153°C.

ANALYSIS:

Calculated for $C_{17}H_{22}N_2$:
80.25%C 8.74%H 11.01%N
Found:
80.12%C 8.89%N 11.06%N

Example 2

1,2,3,4,5,6-Hexahydro-3,6,12-trimethyl-2,6-methanopyrrolo[2,3-j][3]benzazocine-10,11(9H)-dione.

To 46.28 g of a 2:1 molar mixture of 8-amino-1,2,3,4,5,6-hexahydro-3,6,11-trimethyl-2,6-methano-3-benzazocine and 9-amino-1,2,3,4,5,6-hexahydro-3,6,11-trimethyl-2,6-methano-3-benzazocine was added 770 ml of 5% aqueous hydrochloric acid, 950 ml of water, 151.16 g of anhydrous sodium sulfate, and 37.03 g of hydroxylamine hydrochloride. The resulting solution was heated to reflux at which time a solution of 33.29 g of chloral hydrate in 480 ml of water, also heated to reflux, was added. The reaction mixture was then refluxed for 1.2 hours, cooled to 0° C, basified to a pH of 8-9 by the addition of dilute ammonium hydroxide, and extracted with dichloromethane-ethyl acetate (having incorporated therein a small amount of methanol). The extract was washed with brine, dried over anhydrous magnesium sulfate, filtered free of inorganic salts, and concentrated. The concentrate was dissolved in 500 ml of 10% aqueous sulfuric acid and the resulting solution was then warmed for 25-30 minutes at a temperature of 70-75° C. The solution was then poured over ice, basified by the addition of concentrated ammonium hydroxide, and extracted with dichloromethane. The extract was washed with brine, dried over anhydrous magnesium sulfate, filtered, and concentrated to afford a mixture of 1,2,3,4,5,6-hexahydro-3,6,12-trimethyl-2,6-methanopyrrolo[2,3-j][3]-benzazocine-10,11(9H)-dione, 1,2,3,4,5,6-hexahydro-3,6,12-trimethyl-2,6-methano-3-pyrrolo[3,2-i][3]-benzazocine-8,9(10H)-dione, 1,2,3,4,5,6-hexahydro-3,6,12-trimethyl-2,6-methanopyrrolo[3,2-h][3]-benzazocine-7,8(9H)-dione, and 1,2,3,4,5,6- hexahydro-3,6,12-trimethyl-2,6-methanopyrrolo[2,3-i][3]-benzazocine-9,10(8H)-dione. Flash chromatography of the isatin mixture (silica gel; eluting first with a solution of 1-2% triethylamine/0-5% methanol/ethylacetate, and then with 100% methanol) permitted partial separation of the components. The various fractions were then further purified by successive chromatographic separations under conditions as previously described. Trituration of the 1,2,3,4,5,6-hexahydro-3,6,12-trimethyl-2,6-methanopyrrolo[2,3-j]-[3]benzazocine-10,11(9H)-dione fraction with diethylether/pentane and recrystallization from dichloromethane yielded 2.30 g (5%) of product, m.p. 220-222° C (dec.)

ANALYSIS:

Calculated for $C_{17}H_{20}N_2O_2$:
71.79%C 7.10%H 9.85%N
Found:
71.47%C 7.15%H 9.80%N

## Example 3

1,2,3,4,5,6-Hexahydro-3,6,12-trimethyl-2,6-methano-9H-pyrrolo[2,3-j][3]benzazocine

To a stirred slurry of 11.35 g of lithium aluminum hydride and 700 ml of tetrahydrofuran, cooled to 0° C under nitrogen, was added, via cannula, a solution of 8.50 g of 1,2,3,4,5,6-hexahydro-3,6,12-trimethyl-2,6-methanopyrrolo[2,3-j][3]benzazocine-10,11(9H)-dione. The reaction mixture was refluxed for 3 hours, cooled to 0° C, and quenched with 10% aqueous tetrahydrofuran. The aqueous mixture was then dried over anhydrous sodium sulfate, filtered, and concentrated. The concentrate was purified by means of flash chromatography (silica gel; 2% triethylamine/0-3% methanol/ethyl acetate) to yield 2.70 g (35%) of 1,2,3,4,5,6-hexahydro-3,6,12-trimethyl-2,6-methano-9H-pyrrolo[2,3-j][3]benzazocine.

Recrystallization from methanol-pentane afforded the analytical sample, m.p. 224-227° C.

ANALYSIS:

Calculated for $C_{17}H_{22}N_2$:
80.25%C 8.73%H 11.01%N
Found:
79.92%C 8.98%H 11.03%N

## Example 4

1,2,3,4,5,6,9,10-Octahydro-3,6,12-trimethyl-2,6-methano-9H-pyrrolo[2,3-j][3]benzazocine

To a solution of 1.69 of 1,2,3,4,5,6,-hexahydro-3,6,12-trimethyl-2,6-methano-9H-pyrrolo[2,3-j][3]-benzazocine in 25 ml of acetic acid, cooled to 15°C under nitrogen, was added 1.83 g of sodium cyanoborohydride. After stirring for 2 hours at 15-20°C the reaction mixture was quenched with 100 ml of water and basified to a pH of 12 by the addition of 50% aqueous sodium hydroxide.

The aqueous mixture was extracted with dichloromethane/diethyl ether and the combined organic extract was then washed with dilute aqueous sodium bicarbonate and brine, dried over anhydrous potassium carbonate, filtered and concentrated.

The concentrate was purified by flash chromatography (silica gel; 2% triethylamine/0-5% methanol/ethylacetate) to yield 1.50 g (88%) of 1,2,3,4,5,6,10,11-octahydro-3,6,12-trimethyl-2,6-methano-9H-pyrrolo[2,3-j][3]benzazocine.

The free base was dissolved in methanol, treated with ethereal hydrogen chloride and concentrated to precipitate the and corresponding dihydrochloride salt. The salt was redissolved in methanol and precipitated by the addition of diethylether to afford the hygroscopic product 1,2,3,4,5,6,10,11-octahydro-3,6,12-trimethyl-2,6-methano-9H-pyrrolo[2,3-j][3]benzazocine dihydrochloride 3:1 hydrate, mp 309-311°C.

ANALYSIS:

Calculated for $C_{17}H_{26}N_2Cl_2.O.3H_2O$:
60.99%C 8.03%H 8.37%N
Found:
60.69%C 8.07%H 8.22%N

## Example 5

1,2,3,4,5,6,9,10-Octahydro-3,6,12-trimethyl-2,6-methano-8H-pyrrolo[2,3-i][3]benzazocine

To a stirred solution of 5.44 g of 1,2,3,4,5,6-hexahydro-3.6,12-trimethyl-2,6-methano-8H-pyrrolo-[2,3-i]-[3]benzazocine (see Example 1) in 56 ml of acetic acid, cooled to 15°C under nitrogen, was added, 4.13 g of sodium cyanoborohydride. After stirring for 2.25 hours at 15-18°C the reaction mixture was quenched with 107 ml of water and basified to a pH of 12 by the addition of 50% aqueous sodium hydroxide.

The aqueous mixture was extracted with dichloromethane/diethyl ether and the combined organic extract was then washed with brine, dried over anhydrous sodium sulfate, filtered and concentrated. The concentrate was purified by flash chromatography (silica gel; 2% triethylamine/0-4.5% methanol/ethylacetate) to yield 4.38 g (60%) of 1,2,3,4,5,6,9,10-octahydro-3,6,12-trimethyl-2,6-methano-8H-pyrrolo[2,3-i]-[3]benzazocine.

Recrystallization from hexane afforded the analytical sample, m.p. 80-83°C.

ANALYSIS:

Calculated for $C_{17}H_{24}N_2$:
79.62%C 9.45%H 10.93%N
Found:
79.44%C 9.62%H 11.01%N

## Example 6

8-Acetyl-1,2,3,4,5,6,9,10-octahydro-3,6,12-trimethyl-2,6-methano-8H-pyrrolo[2,3-i][3]benzazocine

To a solution of 6.33g of 1,2,3,4,5,6,9,10-octahydro-3,6,12-trimethyl-2,6-methano-8H-pyrrolo[2,3-i][3]-benzazocine, 70 ml of dichloromethane, 0.001g of 4-dimethylaminopyridine and 6.90 ml of triethylamine, cooled to 0°C under nitrogen, was added 2.63 ml of acetyl chloride. After stirring for 0.5 hour at room temperature, the reaction mixture was diluted by the addition of 170 ml of dichloromethane followed by dilute aqueous sodium hydroxide. Upon standing, the mixture separated into aqueous and organic layers. The organic layer was washed with dilute aqueous sodium bicarbonate. The combined aqueous layers were

then back extracted with dichloromethane.

The combined organic layers were washed with brine, dried over anhydrous potassium carbonate, filtered and concentrated. Purification of the concentrate by means of column chromatography (florisil; dichloromethane) followed by washing through a pad of silica gel (5% triethylamine/10% methanol/dichloromethane) afforded a crude product which was triturated with 10-50% dichloromethane/pentane and recrystallized from dichloromethane to yield 2.50g (34%) of 8-acetyl-1,2,3,4,5,6,9,10-octahydro-3,6,12-trimethyl-2,6-methano-8H-pyrrolo[2,3-i][3]benzazocine, m.p. 239.5-242° C.

ANALYSIS:

Calculated for $C_{19}H_{26}N_2O$:
76.45%C 8.80%H 9.39%N
Found:
76.02%C 8.80%H 9.28%N

Example 7

8-Ethyl-1,2,3,4,5,6,9,10-octahydro-3,6,12-trimethyl-2,6-methano-8H-pyrrolo[2,3-i][3]benzazocine

To a solution of 4.1g of 8-acetyl-1,2,3,4,5,6,9,10-octahydro-3,6,12-trimethyl-2,6-methano-8H-pyrrolo[2,3-i][3]benzaocine in 100 ml of tetrahydrofuran, cooled to 0° C under nitrogen, was added 1.04g of lithium aluminum hydroxide. The reaction mixture was warmed to room temperature and then heated at reflux for 1.5 hours. After cooling to room temperature excess lithium aluminum hydride was destroyed by the addition of 10% aqueous tetrahydrofuran. The slurry was basified by the addition of dilute aqueous sodium hydroxide and filtered through a celite pad. Diethyl ether was then added. Upon standing, the mixture separated into aqueous and organic layers.

The aqueous layer was extracted with dichloromethane/diethyl ether. The combined organic layers were washed with brine, dried over anhydrous potassium carbonate, filtered, and concentrated.

The concentrate was twice purified by flash chromatography, (silica gel; 2% triethylamine/0-3% methanol/ethyl acetate) to afford 2.00g (51%) of 8-ethyl-1,2,3,4,5,6,9,10-octahydro-3,6,12-trimethyl-2,6-methano-8H-pyrrolo[2,3-i][3]benzazocine.

Recrystallization from pentane yielded the analytical sample, m.p. 93.5-96° C.

ANALYSIS:

Calculated for $C_{19}H_{28}N_2$:
80.21%C 9.94%H 9.85%N
Found:
80.32%C 9.97%H 9.59%N

Example 8

1,2,3,4,5,6-hexahydro-3,6,12-trimethyl-2,6-methano-10H-pyrrolo[3,2-i][3]benzazocine

To a stirred slurry of 16.03g lithium aluminum hydride in 1000 ml of tetrahydrofuran, cooled to 0° C under nitrogen, was added, via cannula, a solution of 12.00g of 1,2,3,4,5,6-hexahydro-3,6,12-trimethyl-2,6-methanopyrrolo[3,2-i][3]benzazocine-8,9(10H)-dione (see Example 2) in 350 ml of tetrahydrofuran. After refluxing for 3 hours, the reaction mixture was cooled to 0° C and quenched by the addition of 10% aqueous tetrahydrofuran. The mixture then dried over anhydrous sodium sulfate, filtered, and concentrated. The concentrate was purified by means of flash chromatography via a series of separations (silica gel, 2% triethylamine/0-5% methanol/ethyl acetate; alumina, 2% triethylamine/ethyl acetate; silica gel, 2% triethylamine/0-10%-methanol/ethyl acetate) to yield 3.00g (28%) of 1,2,3,4,5,6-hexahydro-3,6,12-trimethyl-2,6-methano-10H-pyrrolo[3,2-i][3]benzazocine.

Recrystallization from dichloromethane-diethyl ether afforded the analytical sample, m.p. 225° - 227° C.

ANALYSIS:

Calculated for $C_{17}H_{22}N_2$:
80.25%C 8.74%H 11.01%N
Found:
79.44%C 9.55%H 10.74%N

## Example 9

1,2,3,4,5,6-hexahydro-3,6,12-trimethyl-2,6-methano-9H-pyrrolo[3,2-j][3]benzazocine.

To a stirred solution 0.42 of 3-ethoxycarbonyl-1,2,3,4,5,6-hexahydro-6,12-dimethyl-2,6-methanopyrrolo-[3,2-h][3]benzazocine-7,8,(9H)-dione (see Example 1) in 15 ml of tetrahydrofuran, cooled to 0 °C under nitrogen, was added 0.56g of lithium aluminum hydride. After refluxing for 3 hours, the reaction mixture was cooled to 0 °C and quenched by the addition of 10% aqueous tetrahydrofuran. The mixture was then dried over anhydrous sodium sulfate, filtered and concentrated.

The above described reaction was repeated utilizing 0.67g of the dione in 70 ml of tetrahydrofuran and adding 0.89 g of lithium aluminium hydride.

Purification of the combined concentrates by means of flash chromatography (silica gel, 2% triethylamine/0-2% methanol/ethyl acetate) afforded 0.36 g (44.5%) of 1,2,3,4,5,6-hexahydro-3,6,12-trimethyl-2,6-methano-9H-pyrrolo[3,2-h][3]benzazocine.

Trituration with dichloromethane-pentane afforded the analytical sample, m.p. 227-229 °C.

ANALYSIS:

Calculated for $C_{17}H_{22}N_2$:
80.25%C 8.74%H 11.01%N
Found:
.79.72%C 9.15%H 11.23%N

REACTION SCHEME A

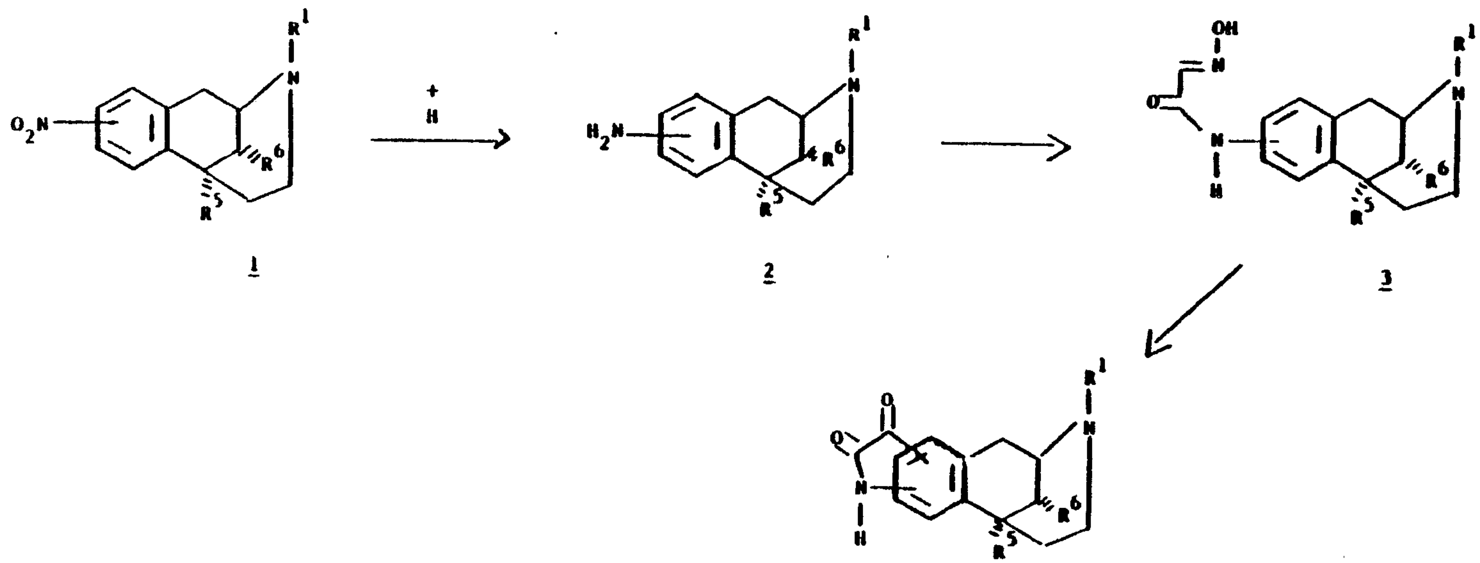


1

2

3

4

wherein $R^1$, $R^5$ and $R^6$ are as hereindefined.

REACTION SCHEME B

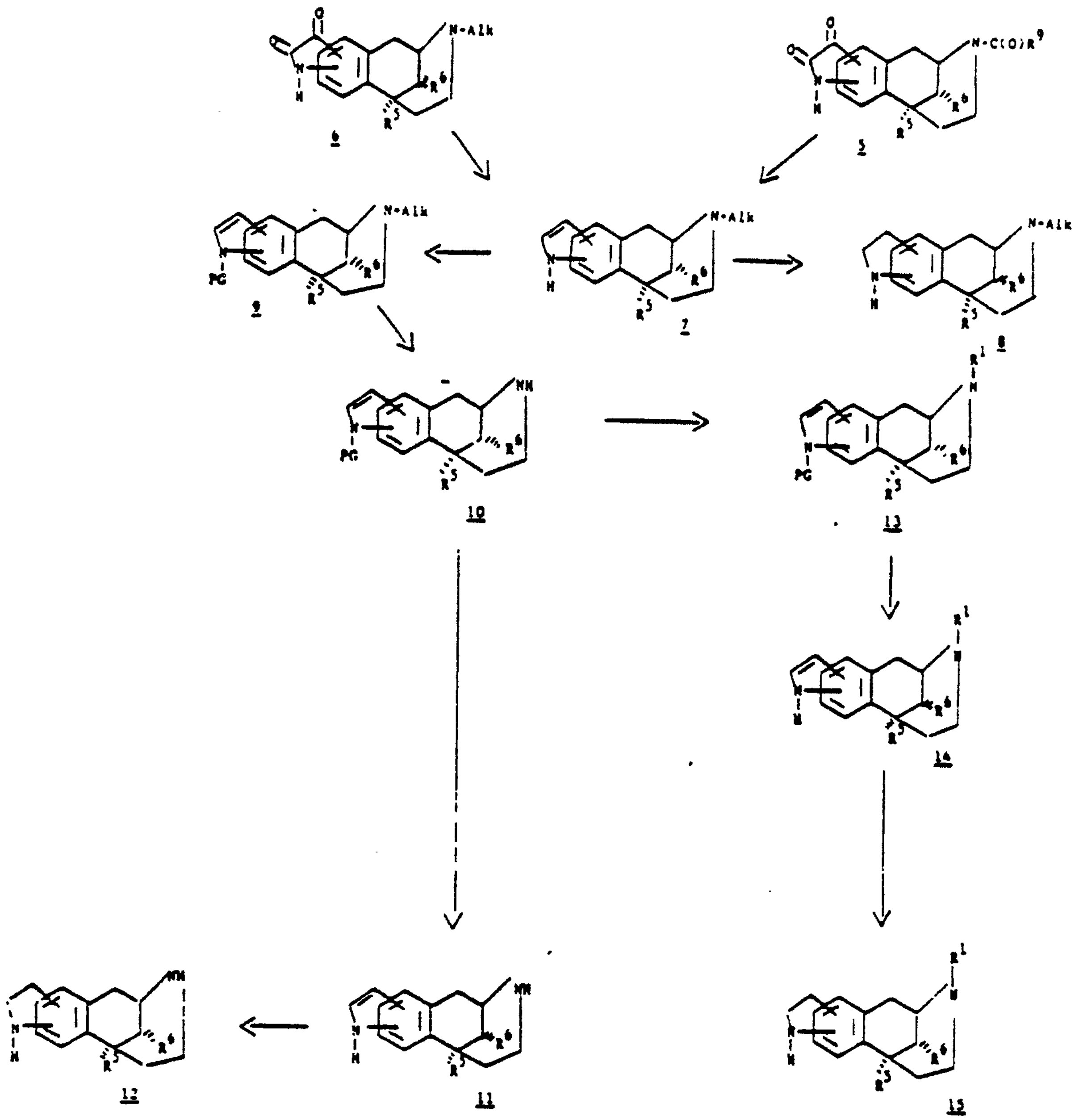

6 5 9 7 8 10 13 14 11 12 15

wherein Alk is loweralkyl or arylloweralkyl; $R^9$ is hydrogen, loweralkyl or loweralkoxy; $R^1$ is loweralkyl, loweralkenyl, cycloalkylloweralkyl or arylloweralkyl; $R^5$ and $R^6$ are independently hydrogen or loweralkyl or taken together are $-(CH_2)_4-$; and PG is a protecting group as hereindefined.

## Claims

1. A compound of the formula I

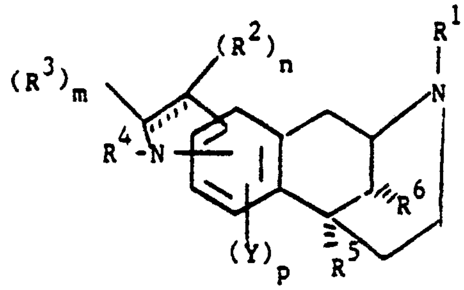

I

wherein $R^1$ is hydrogen, loweralkyl, loweralkenyl, cycloalkylloweralkyl, arylloweralkyl or -C(O)$R^9$ wherein $R^9$ is hydrogen, loweralkyl or loweralkoxy; $R^2$ and $R^3$ are independently loweralkyl, arylloweralkyl, halogen, oxo or -C(O)$R^7$ wherein $R^7$ is hydrogen, loweralkyl, aryl, arylloweralkyl, hydroxy, loweralkoxy, arylloweralkoxy, aryloxy or amino; $R^4$ is hydrogen, loweralkyl, arylloweralkyl or -C(O)$R^8$ wherein $R^8$ is hydrogen, loweralkyl, arylloweralkyl, aryl, loweralkoxy, arylloweralkoxy or aryloxy; $R^5$ and $R^6$ are independently hydrogen or loweralkyl, or taken together are a bivalent radical of the formula $-(CH_2)_4-$; m, n and p are integers independently having values of zero or 1, with the proviso that the sum of m and n only exceeds 1 when at least one of $R^2$ and $R^3$ is linear loweralkyl, hydrogen, or oxo; Y is halogen or loweralkyl; and the dotted line in said compound is an optional bond; and the geometrical isomers, optical antipodes; or pharmaceutically acceptable acid addition salts thereof.

2. A compound as defined in claim 1 wherein m, n and p are each zero.

3. A compound as defined in claim 2 wherein $R^1$, $R^5$ and $R^6$ are each loweralkyl and $R^4$ is hydrogen, loweralkyl or -C(O)$R^8$ where $R^8$ is hydrogen or loweralkyl.

4. A compound as defined in claim 3 wherein $R^4$ is hydrogen.

5. The compound as defined in claim 4 which is 1,2,3,4,5,6-hexahydro-3,6,12-trimethyl-2,6-methano-9H-pyrrolo-[3,2-h][3]-benzazocine, or a pharmaceutically acceptable acid addition salt thereof.

6. The compound as defined in claim 4 which is 1,2,3,4,5,6-hexahydro-3,6,12-trimethyl-2,6-methano-10H-pyrrolo-[3,2-i][3]-benzazocine or a pharmaceutically acceptable acid addition salt thereof.

7. A compound of the formula 3

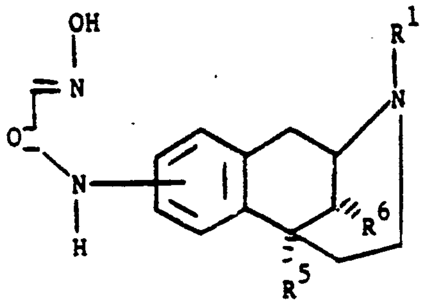

wherein $R^1$ is hydrogen, loweralkyl, loweralkenyl, cycloalkylloweralkyl, arylloweralkyl or -C(O)$R^9$ wherein $R^9$ is hydrogen, loweralkyl or loweralkoxy; and $R^5$ and $R^6$ are independently hydrogen or loweralkyl, or taken together are a bivalent radical of the formula $-(CH_2)_4-$.

8. A compound of the formula 4

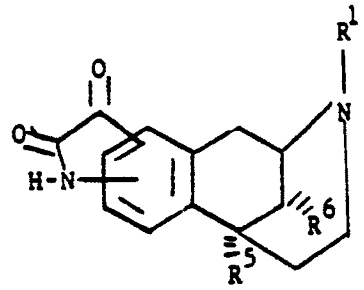

wherein $R^1$ is hydrogen, loweralkyl, loweralkenyl, cycloalkylloweralkyl, arylloweralkyl, or -C(O)$R^9$ wherein $R^9$ is hydrogen, loweralkyl or loweralkoxy; and $R^5$ and $R^6$ are independently hydrogen or loweralkyl; the geometrical isomers; optical antipodes, or pharmaceutically acceptable acid addition salts thereof.

9. A pharmaceutical composition which comprises as the active ingredient a compound as defined in claim 1 together with a suitable carrier therefor.

10. Use of a compound as defined in claim 1 or 8 for the preparation of a medicament having analgesic activity.

11. A process for the preparation of a compound as defined in claim 1 which comprises

a) cyclizing a compound of the formula 3

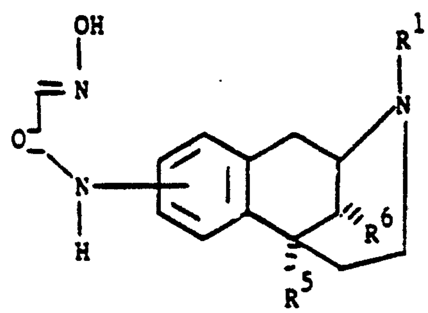

wherein $R^1$, $R^5$ and $R^6$ are as defined, to afford a compound of the formula 4

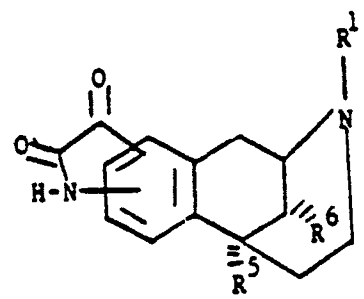

wherein $R^1$, $R^5$ and $R^6$ are as defined,

b) reducing a compound of the formula 4 wherein $R^1$ is loweralkyl or the group -C(O)$R^9$ where $R^9$ is hydrogen, loweralkyl or loweralkoxy to afford a compound of the formula Ia

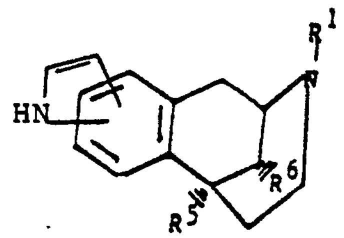

Ia

wherein $R^1$ is loweralkyl, and $R^5$ and $R^6$ are as defined,

c) optionally reducing a compound of the formula Ia where $R^1$ is loweralkyl to afford a compound of the formula Ib

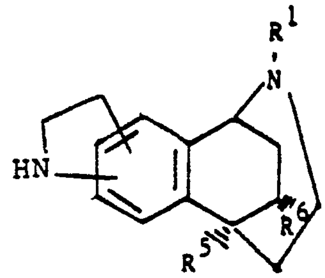

Ib

wherein $R^1$ is loweralkyl and $R^5$ and $R^6$ are as defined,

d) optionally reacting a compound of the formula Ia with an arylloweralkylsulfonyl halide, arylsulfonylhalide or triisopropylsilyltrifluoromethane sulfonate in the presence of a strong base to afford a compound of the formula 9

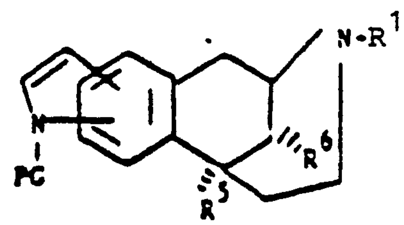

9

wherein $R^1$ is loweralkyl, $R^5$ and $R^6$ are as defined and PG is a protecting group, and

e) subsequently cleaving the 3-loweralkyl group by treating a compound of the formula 9 with an alkyl-, phenyl- or benzylchloroformate and hydrolyzing the resulting carbamate to afford a 3-unsubstituted compound of the formula 10

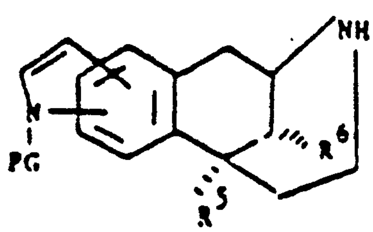

10

f) removing the protecting group in a compound of the formula 10 to afford a compound of the formula Ia wherein $R^1$ and $R^4$ are hydrogen and $R^5$ and $R^6$ are as defined, or

g) treating a compound of the formula 10 with a halide of the formula $R^1Z$ wherein Z is chlorine, bromine or iodine and $R^1$ is loweralkyl, loweralkenyl, cycloalkylloweralkyl, arylloweralkyl or $-C(O)R^9$ where $R^9$ is loweralkyl or loweralkoxy, to afford a compound of the formula 13

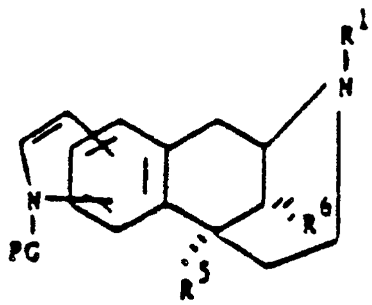

13

and removing the protecting group to afford a compound of the formula Ia where $R^1$ is as hereinbefore defined,

h) optionally reducing a compound of the formula Ia wherein $R^1$ is hydrogen or is as defined in step g) to afford a compound of the formula Ib, where $R^5$ and $R^6$ are as defined above and $R^1$ is hydrogen or is as defined in step g).

Claims for the following Contracting States: ES, GR

1. A process for the preparation of a compound of the formula I

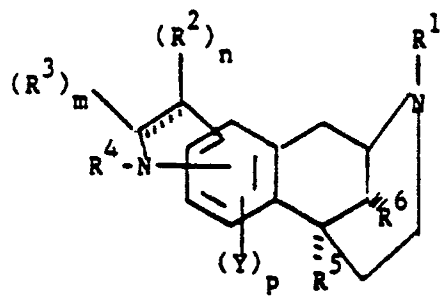

wherein $R^1$ is hydrogen, loweralkyl, loweralkenyl, cycloalkylloweralkyl, arylloweralkyl or $-C(O)R^9$ wherein $R^9$ is hydrogen, loweralkyl or loweralkoxy; $R^2$ and $R^3$ are independently loweralkyl, arylloweralkyl, halogen, oxo or $-C(O)R^7$ wherein $R^7$ is hydrogen, loweralkyl, aryl, arylloweralkyl, hydroxy, loweralkoxy, arylloweralkoxy, aryloxy or amino; $R^4$ is hydrogen, loweralkyl, arylloweralkyl or $-C(O)R^8$ wherein $R^8$ is hydrogen, loweralkyl, arylloweralkyl, aryl, loweralkoxy, arylloweralkoxy or aryloxy; $R^5$ and $R^6$ are independently hydrogen or loweralkyl, or taken together are a bivalent radical of the formula $-(CH_2)_4-$; m, n and p are integers independently having values of zero or 1, with the proviso that the sum of m and n only exceeds 1 when at least one of $R^2$ and $R^3$ is linear loweralkyl, hydrogen, or oxo; Y is halogen or loweralkyl; and the dotted line in said compound is an optional bond; and the geometrical isomers, optical antipodes; or pharmaceutically acceptable acid addition salts thereof, which comprises

a) cyclizing a compound of the formula 3

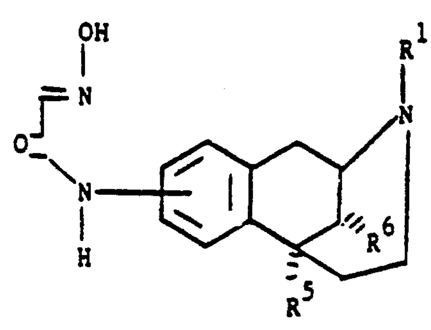

wherein $R^1$, $R^5$ and $R^6$ are as defined, to afford a compound of the formula 4

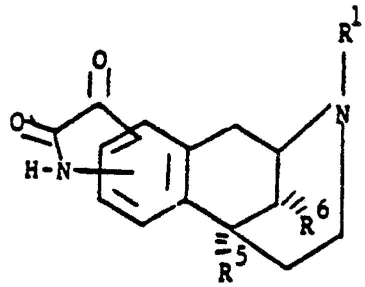

wherein $R^1$, $R^5$ and $R^6$ are as defined,

b) reducing a compound of the formula 4 wherein $R^1$ is loweralkyl or the group $-C(O)R^9$ where $R^9$ is hydrogen, loweralkyl or loweralkoxy to afford a compound of the formula Ia

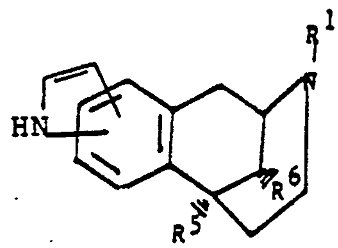

Ia

wherein $R^1$ is loweralkyl, and $R^5$ and $R^6$ are as defined,

c) optionally reducing a compound of the formula Ia wherein $R^1$ is loweralkyl to afford a compound of the formula Ib

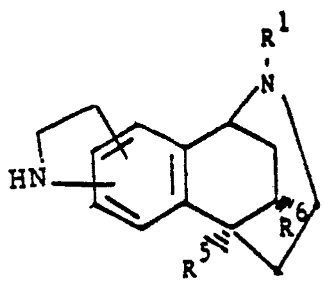

Ib

wherein $R^1$ is loweralkyl and $R^5$ and $R^6$ are as defined,

d) optionally reacting a compound of the formula Ia with an arylloweralkylsulfonyl halide, arylsulfonylhalide or triisopropylsilyltrifluoromethane sulfonate in the presence of a strong base to afford a compound of the formula 9

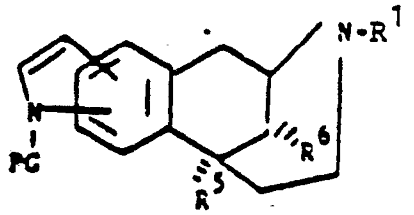

9

wherein $R^1$ is loweralkyl, $R^5$ and $R^6$ are as defined and PG is a protecting group, and

e) subsequently cleaving the 3-loweralkyl group by treating a compound of the formula 9 with an alkyl-, phenyl- or benzylchloroformate and hydrolyzing the resulting carbamate to afford a 3-unsubstituted compound of the formula 10

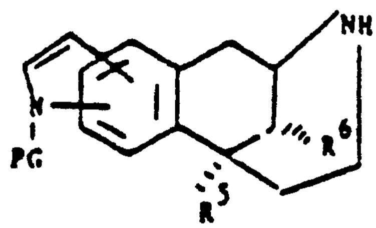

10

f) removing the protecting group in a compound of the formula 10 to afford a compound of the formula Ia wherein $R^1$ and $R^4$ are hydrogen and $R^5$ and $R^6$ are as defined, or

g) treating a compound of the formula 10 with a halide of the formula $R^1Z$ wherein Z is chlorine, bromine or iodine and $R^1$ is loweralkyl, loweralkenyl, cycloalkylloweralkyl, arylloweralkyl or $-C(O)R^9$ where $R^9$ is loweralkyl or loweralkoxy, to afford a compound of the formula 13

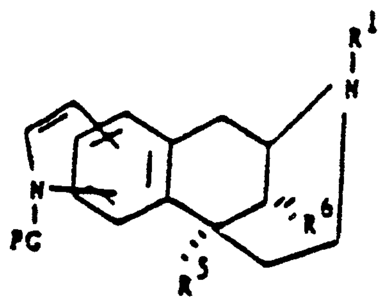

13

and removing the protecting group to afford a compound of the formula Ia where $R^1$ is as hereinbefore defined,

h) optionally reducing a compound of the formula Ia wherein $R^1$ is hydrogen or is as defined in step g) to afford a compound of the formula Ib, where $R^5$ and $R^6$ are as defined above and $R^1$ is hydrogen or is as defined in step g).

2. A process as defined in claim 1 wherein m, n and p are each zero.

3. A process as defined in claim 2 wherein $R^1$, $R^5$ and $R^6$ are each loweralkyl and $R^4$ is hydrogen, loweralkyl or $-C(O)R^8$ where $R^8$ is hydrogen or loweralkyl.

4. A process as defined in claim 3 wherein $R^4$ is hydrogen.

5. A process as defined in claim 4 which is 1,2,3,4,5,6-hexahydro-3,6,12-trimethyl-2,6-methano-9H-pyrrolo-[3,2-h][3]-benzazocine, or a pharmaceutically acceptable acid addition salt thereof is prepared.

6. A process as defined in claim 4 which is 1,2,3,4,5,6-hexahydro-3,6,12-trimethyl-2,6-methano-10H-pyrrolo-[3,2-i][3]-benzazocine or a pharmaceutically acceptable acid addition salt thereof.

7. A process for the preparation of a compound of the formula 3

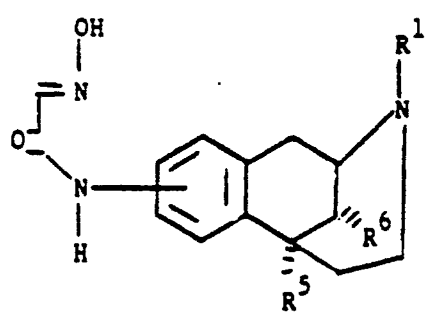

3

wherein $R^1$ is hydrogen, loweralkyl, loweralkenyl, cycloalkylloweralkyl, arylloweralkyl or $-C(O)R^9$ wherein $R^9$ is hydrogen, loweralkyl or loweralkoxy; and $R^5$ and $R^6$ are independently hydrogen or loweralkyl, or taken together are a bivalent radical of the formula $-(CH_2)_4-$, which comprises reacting a compound of the formula

2

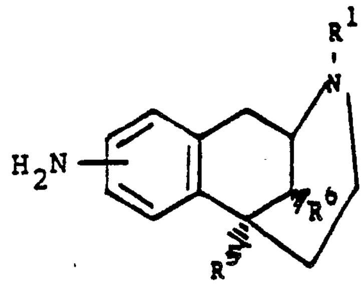

2

where $R^1$, $R^5$ and $R^6$ are defined, with hydroxylamine hydrochloride and chloral hydrate.

8. A process for the preparation of a compound of the formula 4

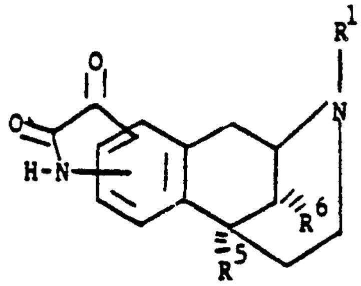

4

wherein $R^1$ is hydrogen, loweralkyl, loweralkenyl, cycloalkylloweralkyl, arylloweralkyl, or -C(O)$R^9$ wherein $R^9$ is hydrogen, loweralkyl or loweralkoxy; and $R^5$ and $R^6$ are independently hydrogen or loweralkyl; the geometrical isomers; optical antipodes, or pharmaceutically acceptable acid addition salts thereof, which comprises cyclizing a compound of the formula 3 as defined in claim 7 by treatment with a mineral acid or phosphoric acid.

9. Use of a compound as defined in claim 1 or 8 for the preparation of a medicament having analgesic activity.